# EUROPÄISCHE PATENTSCHRIFT

(11) **EP 1 476 531 B1**
(45) Veröffentlichungstag und Bekanntmachung des Hinweises auf die Patenterteilung: **28.03.2012**
(21) Anmeldenummer: 02792962.9
(22) Anmeldetag: 11.12.2002
(51) Int. Cl.: A61K 8/11, A61K 8/37, A61K 8/92, A61Q 13/00, B01J 13/12, C11D 3/22, C11D 17/00, B01J 13/02, A61L 9/01, A61L 9/12, A61L 9/04, A61K 9/16, C11D 3/50, A61K 8/73

(54) **POLYMERE DUFTKAPSELN UND IHRE HERSTELLUNG**
SCENTED POLYMER CAPSULES AND THE PRODUCTION THEREOF
CAPSULES POLYMERES PARFUMEES ET LEUR PRODUCTION

(30) Priorität: 20.12.2001 DE 10163142
(43) Veröffentlichungstag der Anmeldung: 17.11.2004
(73) Patentinhaber: Henkel AG & Co. KGaA, 40589 Düsseldorf (DE)
(72) Erfinder: SCHMIEDEL, Peter, 40599 Düsseldorf (DE); BECKER, Axel, 64319 Pfungstadt (DE); HARDACKER, Ingo, 46499 Hamminkeln (DE); FERENCZ, Andreas, 40223 Düsseldorf (DE)
(86) Internationale Anmeldenummer: PCT/EP2002/014050
(87) Internationale Veröffentlichungsnummer: WO 2003/054130

(56) Entgegenhaltungen:
- EP-A- 0 305 139
- EP-A- 1 061 124
- WO-A-00/32043
- WO-A-97/47288
- DE-A- 2 523 586
- US-A- 3 691 090
- US-A- 4 209 417
- US-A- 4 548 764
- US-A- 5 051 305

## Beschreibung

Die vorliegende Erfindung betrifft Verfahren zur Herstellung duft- bzw. riechstoffbeladener Polymerkapseln.

Für viele Produkte ist es heute aus ästhetischen Gründen reizvoll, aktive Bestandteile, wie beispielsweise Duft- und/oder Riechstoffe oder Aroma-, Parfüm-, Pflege- und Silikonöle, separat in abgegrenzter Form zuzugeben, z. B. in Form von Kapseln, Kugeln, Tropfen oder als zweite Phase. Neben den ästhetischen Vorteilen zeigen solche räumlichen Abgrenzungen vielfach auch Stabilitäts- und Formuliervorteile.

Aktiv- und/oder Wirkstoffe, z. B. Duft- und Riechstoffe oder Öle wie etherische Öle und Aroma-, Parfüm-, Pflege- oder Silikonöle, die in kosmetischen Produkten oder in Wasch-und Reinigungsmitteln eingesetzt werden, verlieren häufig schon bei der Lagerung oder aber direkt bei der Anwendung ihre Aktivität. Manche dieser Stoffe können auch eine zur Verwendung nicht ausreichende Stabilität besitzen oder störende Wechselwirkungen mit anderen Produktbestandteilen verursachen. Daher ist es von Interesse, solche Substanzen kontrolliert und am gewünschten Einsatzort mit maximaler Wirkung einzusetzen.

Des weiteren ergibt sich beispielsweise bei Reinigungsmitteln, wie z.B. Universalreinigern, Badreinigern und insbesondere Fußbodenreinigem, häufig das Problem, daß deren Duft bei ihrer Anwendung oder unmittelbar danach sehr intensiv ist, aber der Duft nach kurzer Zeit jedoch kaum mehr wahrnehmbar ist. Ähnliche Probleme ergeben sich bei Textilien nach deren Wäsche: Der anfängliche intensive Duft klingt relativ rasch ab.

Aus diesem Grunde werden die Aktiv- und/oder Wirksubstanzen den Produkten oftmals in räumlich abgegrenzter, geschützter Form zugesetzt. Häufig werden empfindliche Substanzen in Kapseln verschiedener Größen eingeschlossen, auf geeigneten Trägermaterialien adsorbiert oder chemisch modifiziert. Die Freisetzung kann dann mit Hilfe eines geeigneten Mechanismus aktiviert werden, beispielsweise mechanisch durch Scherung, oder diffusiv direkt aus dem Matrixmaterial erfolgen.

Oftmals sind auch Systeme wünschenswert, die sich als Verkapselungs-, Transport- oder Darreichungsvehikel - oft synonym auch als "Delivery-Systeme" oder "Carrier-Systeme" bezeichnet - eignen.

Es existieren bereits zahlreiche kommerzielle Delivery-Systeme, die auf porösen Polymerpartikeln oder Liposomen basieren (z. B. Mikrosponges^{®} von der Firma Advanced Polymer Systems oder aber Nanotopes^{®} von der Firma Ciba-Geigy, siehe hierzu B. Herzog, K. Sommer, W. Baschong, J. Röding "Nanotopes™: A Surfactant Resistant Carrier System" in SÖFW-Journal, 124. Jahrgang 10/98, Seiten 614 bis 623).

Der Nachteil dieser herkömmlichen, aus dem Stand der Technik bekannten Delivery-Systeme besteht darin, daß sie nur ein geringes Beladungspotential aufweisen, die Partikelgröße der Polymerkugeln meist im Bereich von einigen Mikrometem bis einigen 100 pm liegt und eine Verkapselung der Wirksubstanzen in der Regel nicht in situ erfolgen kann. Die Modifizierung der Kapseloberflächen ist nicht möglich bzw. sehr aufwendig. Liposome besitzen außerdem eine für viele Anwendungen ungenügende Stabilität. Ein weiterer Nachteil dieser herkömmlichen Systeme besteht darin, daß eine Freisetzung von Wirksubstanzen am Ort der spezifischen Anwendung häufig nicht gesteuert werden kann.

Weiterhin werden bei der Herstellung von duft- und/oder riechstoffbeladenen Kapseln nach dem Stand der Technik oftmals störende oder toxische, schlecht riechende oder aggressive Bestandteile in die Formulierung mit eingebracht. Oft werden die Verkapselungen unter aggressiven, die zu verkapselnden Aktiv- bzw. Wirkstoffe belastenden Bedingungen (hohe Temperaturen, lange Reaktionszeiten, Auftreten freier Radikale etc.) durchgeführt.

Die EP 0 397 245 A2 beschreibt ein Verfahren zur Herstellung von Parfümteilchen, bei dem ein polymeres Trägermaterial in der Schmelze mit einem Parfüm versetzt wird und die daraus entstehende Schmelze unter Abkühlung mit flüssigem Stickstoff verfestigt wird. Der entstehende Feststoff wird anschließend gemahlen, bis die Parfümteilchen die gewünschte Größe aufweisen. Ein Nachteil dieses Verfahrens ist die Beschränkung auf Trägermaterialien, die in einem mit dem Parfüm verträglichen Bereich schmelzen. Aufgrund des Schmelzvorgangs bei der Herstellung weisen die resultierenden Parfümteilchen eine hohe Dichte auf, was aufgrund der kompakten Matrix aus polymerem Trägermaterial nur eine geringfügige, nichtsteuerbare Abgabe des Parfüms ermöglicht. Des weiteren ist aufgrund der kompakten Matrix nur eine geringe Beladung mit Parfümstoffen möglich. Eine Modifizierung des polymeren Trägermaterials zur Beeinflussung der Substantivität der Kapseln ist nur unter großem Aufwand durch Modifikation des Polymermaterials möglich und ist aber gleichzeitig durch die Erfordemis der Schmelzbarkeit bei relativ geringen Temperaturen beschränkt.

Die deutsche Patentschrift DE 35 38 429 C2 beschreibt ein Verfahren zur Herstellung eines geschmacksmaskierten Pulvers mit gesteuerter Freisetzung zur Verwendung in eßbaren pharmazeutischen Zusammensetzungen. Zur Herstellung der Kapseln wird ein Polymer in einem Lösemittel gelöst, mit einem aktiven Bestandteil versetzt und dispergiert. Das Lösemittel wird anschließend wieder entfernt. Die nach diesem Verfahren hergestellten Pulver eignen sich nicht zur Verwendung in Wasch- und Reinigungsmitteln.

Ein Verfahren zur Herstellung von mit Arzneimitteln beladenen Polymerkapseln ist bekannt aus R. Bodmeier et al., "Spontaneous formation of drug-containing acrylic nanoparticles", J. Microencapsulation, 1991, Bd. 8, Nr. 2, Seiten 161 bis 170. Dabei wird das Arzneimittel in einem wassermischbaren organischen Lösemittel gelöst, mit einem speziell ammoniumfunktionalisierten Acrylpolymer (Eudragit^{®} RS oder RL 100) versetzt und in Wasser überführt. Dabei bildet sich eine gewünschte Dispersion von Polymerkapseln in dem organischen Lösemittel. Dieses Verfahren ist allerdings auf die Verwendung der speziellen ammoniumfunktionalisierten Acrylpolymeren beschränkt, weil nur diese Polymere genau den benötigten ionischen Charakter bei gleichzeitiger Wasserunlöslichkeit aufweisen, um ein Verklumpen der Polymerkapseln zu vermeiden.

US 5,051,305 beschreibt ein Verkapselungsverfahren unter Einsatz eines Koazervationshilfsmittels, wie z.B. Gum Arabicum oder Natriumpolyphosphat, sowie eines Vernetzers, wie z.B. Glutaraldehyd.

EP 1 061 124 A1 beschreibt ein Verfahren zur Reduktion des Duftverlustes in einem Substrat durch Einsatz einer Duftmatrix und eines Textilconditioners.

US 4,209,417 beschreibt ein Gießverfahren zur Herstellung von Parfümpartikeln.

US 3,691,090 beschreibt ein Verfahren zur Herstellung von Kapseln, bei welchem mit wässrigen Lösungen anorganischer Salze gearbeitet wird.

DE 25 23 586 A1 beschreibt ein Fällverfahren zur Herstellung von Mikrokapseln, bei welchem allerdings keine Tenside eingesetzt werden.

WO 00/32043 A1 beschreibt ein Verfahren zur Herstellung von Polymerkügelchen, bei welchem wasserlösliche Polymere eingesetzt werden.

US 4,548,764 beschreibt Hohlstrukturen, welche im Innern Polymerpartikel enthalten, die aus einem Polymer und einer funktionellen Flüssigkeit bestehen, welche ein Parfümöl sein kann.

EP 0 305 139 A1 beschreibt Polymerpartikel, welche z.B. Parfümöle enthalten, wobei die Partikel über Emulsionspolymerisation hergestellt werden.

WO 97/47288 A1 beschreibt verkapselte Riechstoffe auf Basis bestimmter Acrylsäurecopolymere.

Eine Aufgabe der vorliegenden Efindung ist somit die Bereitstellung eines Matrix- bzw. Depotsystems in Form von Polymerkapseln mit gegenüber dem Stand der Technik verbesserten Eigenschaften, das insbesondere zur Verkapselung duftaktiver Komponenten geeignet ist, sowie entsprechender Herstellungsverfahren.

Eine weitere Aufgabe der vorliegenden Erfindung besteht darin, ein System zur Verfügung zu stellen, das insbesondere bei der Verwendung in Wasch- oder Reinigungsmitteln einen langanhaltenden Duft erzeugt, ohne daß der Duft unmittelbar nach der Anwendung des Produktes unangenehm intensiv sein sollte.

Eine weitere Aufgabe der vorliegenden Erfindung ist die Bereitstellung von Duftkapseln mit Langzeiteffekt ("sustained release") zur Freisetzung insbesondere von duft- und/oder riechaktiven Substanzen. Diese Duftkapseln sollen sich dabei insbesondere für die Verwendung in Wasch- und Reinigungsmitteln eignen.

Gegenstand der Erfindung ist ein Verfahren zur Herstellung von duft- und/oder riechstoffbeladenen Matrix- und/oder Depotsystemen in Form von Polymerkapseln mit mindestens einer duftaktiven Komponente, mit folgenden Verfahrensschritten:
(a) Bereitstellung einer Lösung oder Dispersion, die mindestens ein nicht wasserlösliches Polymer, welches in wässriger Phase zu weniger als 10% löslich ist, und mindestens eine duftaktive Komponente, ausgewählt aus der Gruppe von Duft- und/oder Riechstoffen, Duft-und/oder Riechstoffzubereitungen und Ölen wie etherischen Ölen, Aromaölen, Parfümölen, enthält, mit einem mit Wasser mischbarem organischen Löse- oder Dispersionsmittel;
(b) Eintragung der unter (a) hergestellten Mischung in Wasser oder in eine wässrige Lösung, in Gegenwart eines ionischen Tensids, so dass man duftbeladene Polymerkapseln in wässriger Dispersion erhält;
(c) gegebenenfalls Abtrennung der auf diese Weise erhaltenen Polymerkapseln, wobei die resultierenden Polymerkapseln eine Oberflächenladung aufweisen.

Das Verfahren ermöglicht Polymerkapseln als duft- bzw. riechstoffbeladene Matrix-und/oder Depotsysteme, die mindestens ein polymeres Träger- oder Matrixmaterial auf Basis von insbesondere wasserunlöslichen Cellulosen und/oder Cellulosederivaten umfassen, die mittlere Molekulargewichte von mehr als 30.000, vorzugsweise mehr als 35.000, besonders bevorzugt mehr als 40.000, aufweisen, wobei die Polymerkapsein außerdem mindestens eine duftaktive Komponente enthalten. Diese duftaktive Komponente kann insbesondere aus der Gruppe von Duft- und/oder Riechstoffen, Duft- und/oder Riechstoffzubereitungen und Ölen wie etherischen Ölen, Aromaölen, Parfümölen, Pflegeölen und Silikonölen ausgewählt sein. Darüber hinaus enthalten diese Polymerkapseln mindestens ein Tensid. Falls als Polymermaterial ein Cellulosederivat verwendet wird, so kann dieses beispielsweise ausgewählt sein aus der Gruppe von Alkylcellulosen, insbesondere Ethylcellulose, und Celluloseestern, insbesondere Celluloseacetat und Celluloseacetatbutyrat.

Das Verfahren ermöglicht Polymerkapseln als duft- und/oder riechstoffbeladene Matrix-und/oder Depotsysteme, die mindestens ein polymeres Träger- oder Matrixmaterial auf Basis von insbesondere im wesentlichen wasserunlöslichen Polymeren mit einer mittleren Molekularmasse von mehr als 30.000, insbesondere von mehr als 35.000, vorzugsweise von mehr als 40.000, umfassen. Dabei enthalten diese Polymerkapseln mindestens eine duftaktive Komponente, die vorzugsweise aus der Gruppe von Duft- und/oder Riechstoffen, Duft- und/oder Riechstoffzubereitungen und Ölen wie etherischen Ölen, Aromaölen, Parfümölen, Pflegeölen und Silikonölen ausgewählt sein kann. Ferner enthalten diese Polymerkapseln mindestens ein Tensid. Das Polymer kann ein nichtionisches oder ein ionisches Polymer sein. Das Polymer kann darüber hinaus ein geblocktes und/oder statistisches Comonomer umfassen. Das Polymer kann insbesondere ausgewählt sein aus der Gruppe von Polyalkylenen wie Polybutadienen, Poly(butadien-co-acrylnitrilen), Polyisobutenen, Polyamiden, Polystyrolen, Polyisoprenen, Polycarbonaten, Polyestem, Polyacrylaten, Polymethacrylaten, Polyurethanen, Polydimethylitakonaten, Polydiamylfumaraten, Polybenzylvinylethern, Polyvinylalkoholen, Polyallylalkoholen, Polyvinylformalen, Polyvinylbutyralen, Poly(2-vinyl-4,7-dihydro-1,3-dioxepinen), Polyvinylmethylketonen, Polymethylisopropylketonen, Polyvinylpivalaten, Polyvinylacetylacetaten, Poy(p-formylstyrolen), Poly(2-vinylpyridinen), Polydimethylfulvenen, Poly(carbonyl-1-furfuryltrimethylenen), Polytetrahydrofuranen, Polyglutardialdehyden, Polyoxycarbonyloxyhexamethylenen, Polyethylenadipaten, Polyvinylalkoholacetaten, Polyvinylbutyralacetalen, Polymilchsäuren und Polyoxymethylhexadecylsilylenen. Das Polymer kann ionisch modifiziert sein. Vorzugsweise erfolgt diese ionische Modifizierung durch ionische Gruppen (z. B. Ammonium- bzw. Carboxylatfunktionen), durch Pfropfung bzw. durch Copolymerisation mit geeigneten ionischen Comonomeren.

Das Verfahren ermöglicht Polymerkapseln als duft- bzw. riechstoffbeladene Matrix-und/oder Depotsysteme, die mindestens ein polymeres Träger- oder Matrixmaterial auf Basis eines ionischen Polymers umfassen. Im allgemeinen betragen die mittleren Molekulargewichte mehr als 30.000, vorzugsweise mehr als 35.000, besonders bevorzugt mehr als 40.000. Die Polymerkapseln umfassen zusätzlich mindestens eine duftaktive Komponente, die aus der Gruppe von Duft- und/ oder Riechstoffen, Duft- und/oder Riechstoffzubereitungen und Ölen wie etherischen Ölen, Aromaölen, Parfümölen, Pflegeölen und Silikonölen ausgewählt sein kann. Diese Polymerkapseln können außerdem mindestens ein Tensid enthalten. Das Polymer kann ein geblocktes bzw. statistisches Comonomer umfassen. Darüber hinaus kann das Polymer ein ionisches Polymer bzw. ionisch modifiziert sein. Diese Modifikation kann beispielsweise durch ionische Gruppen, insbesondere Ammonium- und/oder Carboxylatfunktionen, durch Pfropfung und/oder durch Copolymerisation mit geeigneten ionischen Comonomeren erfolgen. Bei einer bevorzugten Variante kann das ionische Polymer ein ammoniumfunktionalisiertes (Meth-)Acrylat-Copolymer, vorzugsweise ein Poly(ethylen-methylmethacrylat-trimethylammo-niumethylmethacrylat)-Copolymer, sein, wobei die stöchiometrischen Verhältnisse der einzelnen Monomereinheiten in dem Poly(ethylen-methylmethacrylat-trimethylammoniumethylmethacrylat)-Copolymer 1 : 2 : 0,1 oder 1 : 2 : 0,2 (z. B. Eudragit^{®} RS und RL 100) betragen können.

Für alle drei erfindungsgemäß herstellbaren Polymerkapseln in Betracht kommende Duft- bzw. Riechstoffe und/oder Duft- bzw. Riechstoffzubereitungen sind natürliche oder synthetische Duft- und/oder Riechstoffe bzw. -zubereitungen. Als Duft- bzw. Riechstoffe können insbesondere Parfümöle oder Mischungen von Parfümölen verwendet werden, die üblicherweise in Wasch- und Reinigungsmitteln oder Kosmetika eingesetzt werden. So können als Parfümöle bzw. Duftstoffe einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z. B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat, Benzylsalicylat, Cyclohexylsalicat, Floramat, Melusat und Jasmacyclat. Zu nennen sind hier auch die Ester von Duftalkoholen mit anorganischen Säuren oder organischen Säuren, wie sie im Stand der Technik offenbart werden. Zu den Ethern zählen beispielsweise Benzylethylether und Ambroxan, zu den Aldehyden z. B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon. Zu den Alkoholen zählen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden Mischungen verschiedener Riechstoffe verwendet, die so aufeinander abgestimmt sind, daß sie gemeinsam eine ansprechende Duftnote erzeugen. Geeignete Parfümöle können auch natürliche Riechstoffgemische enthalten, wie sie aus pflanzlichen Quellen zugänglich sind, z.B. Pine-, Citrus-, Jasmin-, Patchouly-, Rosen- oder Y-lang-Ylang-Öl. Ebenfalls geeignet sind Muskateller, Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl. Weiterhin seien als natürliche Duftkomponente genannt: Extrakte von Blüten (Lilie, Lavendel), Stengeln und Blättern (Geranium, Petitgrain), Früchten (Anis, Koriander, Kümmel, Muskat), Fruchtschalen (Bergamotte, Zitrone, Orangen), Wurzeln (Macis, Angelica, Sellerie, Kardamom, Costus, Iris, Calmus, Vetiver), Hölzern (Guajak-, Zedern-, Rosenholz), Kräutern und Gräsern (Estragon, Lemongras, Thymian), Nadeln und Zweigen (Fichte, Tanne, Kiefer, Latschen), Harzen und Balsamen (Galbanum, Elemi, Benzoe, Myrrhe, Olibanum, Opoponax, Labdanum). Weiterhin kommen tierische Rohstoffe in Frage, wie beispielsweise Zibet und Castoreum. Weitere Verbindungen, die noch als Riechstoffe verwendet werden können, sind: Nitrile, Sulfide, Oxime, Acetale, Ketale, Säuren, Schiffsche Basen, heterocyclische Stickstoffverbindungen (Indol, Chinölin) Pyrazine, (anthranilate) Amine, Amide, halogenorganische Verbindungen (Roseacetat), nitrierte Verbindungen (Nitromoschus), heterocyclische Schwefelverbindungen (Thiazole) und heterocyclische Sauerstoffverbindungen (Epoxide). Beispiele für Parfümöle, die besonders bevorzugt in Wasch- und Reinigungsmitteln und Kosmetika verwendet werden, sind: Orangenöl, insbesondere süßes Orangenöl, Geraniol, Rosenöl, Lilial, α-Hexylzimtaldehyd, Iso E Super, α-Amylzimtaldehyd, Propidyl, Hexylsalicylat, Cedramber, Myraldylacetat, PTBCA 25 cis (Cyclohexanol, 4-(1,1-Dimethylethyl)acetate), Linalool, Tetrahydrolinaool, Citronellol, Aldehyd C12, Troenan, Floramat, Diphenylether, Isoraldein 70, Cyclohexylsalicyclat, Sandelice, Boisambrene Forte. Zu weiteren Einzelheiten bezüglich Duft.- bzw. Riechstoffen und/oder -zubereitungen wird verwiesen auf P. Kraft et al. "Allerlei Trends: die neuesten Entwicklungen in der Riechstoffchemie" in Angew. Chem. 2000, 112, 3106 - 3138, dessen Inhalt hiermit durch Bezugnahme eingeschlossen ist.

Die erfindungsgemäß herstellbaren Polymerkapseln können zusätzlich Wasser enthalten. Ihre mittlere Teilchengröße kann in großen Bereichen variieren; die mittlere Teilchengröße kann im allgemeinen etwa 50 nm bis etwa 500 µm, insbesondere etwa 100 nm bis etwa 250 µm, vorzugsweise etwa 200 nm bis etwa 100 µm, betragen. Auch der Gehalt an duftaktiver(n) Komponente(n) in den erfindungsgemäß herstellbaren Polymerkapsein kann in großen Bereichen variieren; er beträgt im allgemeinen 1 Gew.% bis 60 Gew.%, vorzugsweise 10 Gew.-% bis 50 Gew.-%, wobei diese Gewichtsangaben auf die Polymerkapseln bezogen sind. Der Gehalt an Tensid in den erfindungsgemäß herstellbaren Polymerkapsein kann ebenfalls in großen Bereichen variieren; er beträgt im allgemeinen 0,01 Gew.% bis 50 Gew.-%, bevorzugt ist allerdings ein Tensidgehalt von 0,1 Gew.% bis 40 Gew.%, wobei die Gewichtsangaben auf die erfindungsgemäß herstellbaren Polymerkapseln bezogen sind.

Unter Polymerkapseln versteht man erfindungsgemäß insbesondere Depot- bzw. Matrixkapseln in Form von an Flüssigkeiten reichen dispersen Systemen aus mindestens einem Polymer und mindestens einer duftaktiven Komponente. Dabei meint der Begriff Kapseln erfindungsgemäß keine Core/Shell-Strukturen (Kern/Hülle-Strukturen), sondern vielmehr Matrix- bzw. Depotpartikel. Diese erfindungsgemäß herstellbaren Matrix- und/oder Depotstrukturen bestehen nicht aus einer herkömmlichen Kern/Hülle-Struktur, sondern aus einem System, das aufgrund physikalischer und/oder chemischer Wechselwirkungen, beispielsweise Netzwerkbildung, ausgebildet sein kann. Bei den erfindungsgemäß herstellbaren Kapseln handelt es sich insbesondere um formstabile, leicht deformierbare Partikel. Im vorliegenden Fall können diese Polymerkapseln teilchenförmige, insbesondere schwammartige Strukturen aus Polymer und duftaktiver Komponente ausbilden. Dabei kann die duftaktive Komponente insbesondere in homogener Verteilung über das Polymer vorliegen. Je nach Kompatibilität (Verträglichkeit) von Duftkomponente einerseits und Polymer andererseits erhält man einphasige oder zweiphasige Kapselsysteme. Verwendet man beispielsweise eine Duftkomponente, die mit dem Polymer nicht verträglich ist, erhält man nach Auftragung der Kapseln zu Filmen einen trüben, mikroskopisch zweiphasigen Film. Ist dagegen die Duftkomponente mit dem Polymer verträglich bzw. mischbar erhält man nach Auftragung der Kapseln zu Filmen einen klaren, homogenen, mikroskopisch einphasigen Film, d. h. die Duftkomponente ist dann wie ein Weichmacher homogen in das Polymer eingelagert.

Die in den erfindungsgemäß herstellbaren Polymerkapseln vorhandenen Polymere und/oder duftaktive(n) Komponente(n) sind im wesentlichen wasserunlöslich oder zumindest in wäßriger Phase nur schwer löslich sein. Vorzugsweise sind die Polymere und/oder die duftaktive(n) Komponenten in einem solchen Fall in wäßriger Phase zu weniger als 10 %, vorzugsweise zu weniger als 5 %, insbesondere zu weniger als 1 %, löslich. Wäßrige Phase im Sinn der vorliegenden Erfindung meint auch solche Systeme, die beträchtliche Mengen an organischen Lösemitteln enthalten können, wobei jedoch der Wassergehalt nicht weniger als 50% betragen sollte.

Die erfindungsgemäß herstellbaren Polymerkapseln enthalten ionische, d. h. kationische oder anionische, Tenside (grenzflächenaktive Substanzen). Je nach gewünschter Verwendung der Polymerkapseln werden unterschiedliche Tenside verwendet, um die Substantivität der resultierenden Kapseln zu steuern bzw. für die jeweilige Anwendung maßzuschneidern. So werden z. B. insbesondere kationische Tenside für die erfindungsgemäß herstellbaren Polymerkapseln zum Einsatz in Weichspülmitteln verwendet, da positiv geladene bzw. kationisch modifizierte Kapseln sich leicht auf die negativ geladenen Fasern von Kleidungsstücken aufziehen lassen und somit eine gute Substantivität gegenüber diesen Fasern aufweisen. Erfindungsgemäß geeignete kationische Tenside können dabei insbesondere ausgewählt sein aus der Gruppe von quartären Ammoniumverbindungen wie Dimethyldistearylammoniumchlorid (CTMA-CI), Dodecyltrimethylammoniumbromid, Didodecyldimethylammoniumbromid, Tridodecylmethylammoniumbromid; Tetradodecylammoniumbromid; Esterquats, insbesondere quaternierten Fettsäuretrialkanolaminestersalzen; Salzen langkettiger primärer Amine quaternären Ammoniumverbindungen wie Hexadecyltrimethylammoniumchlorid; Cetrimoniumchlorid oder Lauryldimethylbenzylammoniumchlorid. Demgegenüber eignen sich anionische Tenside vorzugsweise zur Verwendung in den erfindungsgemäßen Polymerkapseln zum Einsatz in Wasch- und Reinigungsmitteln. Erfindungsgemäß geeignete anionsiche Tenside können beispielsweise ausgewählt sein aus der Gruppe von Seifen; Alkylbenzolsulfonaten; Alkansulfonaten; Olefinsulfonaten; Alkylethersulfonaten; Glycerinethersulfonaten; α-Methylestersulfonaten; Sulfofettsäuren; Alkylsulfaten wie Natriumdodecylsulfat (SDS); Fettalkoholethersulfaten; Glycerinethersulfaten; Fettsäureethersulfaten; Hydroxymischethersulfaten; Monoglycerid(ether)sulfaten; Fettsäureamid(ether)sulfaten; Mono- und Dialkylsulfosuccinaten; Mono-und Dialkylsulfosuccinamaten; Sulfotriglyceriden; Amidseifen; Ethercarbonsäuren und deren Salzen; Fettsäureisothionaten; Fettsäuresarcosinaten; Fettsäuretauriden; N-Acylaminosäuren wie Acyllactylaten, Acyltartraten, Acylglutamaten und Acylaspartaten; Alkyloligoglucosidsulfaten; Proteinfettsäurekondensaten, insbesondere pflanzlichen Produkten auf Weizenbasis; Alkyl(ether)phosphaten. Insbeondere im Fall der Verwendung von ionischen Polymeren in den erfindungsgemäß herstellbaren Polymerkapseln kann auch ein nichtionisches Tensid in den Polymerkapseln enthalten sein. Grundsätzlich ist es auch bei Verwendung nichtionischer Polymere möglich, nichtionische Tenside, insbesondere zusätzlich zu ionischen Tensiden, einzusetzen. Die nichtionischen Tenside können beispielweise ausgewählt sein aus der Gruppe von (i) nichtpolymeren nichtionischen Tensiden wie alkoxylierten, vorzugsweise ethoxylierten Fettalkoholen, Alkylphenolen, Fettaminen und Fettsäureamiden; alkoxylierten Triglyceriden, Mischethern und Mischformalen; gegebenenfalls partiell oxidierten Alk(en)yloligoglykosiden; Glucoronsäurederivaten; Fettsäure-N-alkylglucamiden; Proteinhydrolysaten, insbesondere alkylmodifizierten Proteinhydrolysaten; niedermolekularen Chitosanverbindungen; Zuckerestem; Sorbitanestern; Aminoxiden; und (ii) polymeren nichtionischen Tensiden wie Fettalkoholpolyglykolethern; Alkylphenolpolyglykolethem; Fettsäurepolyglykolestern; Fettsäureamidpolyglykolethern; Fettaminpolyglykolethern; Polyolfettsäureestern; Polysorbaten. Durch die Auswahl und Kombination von ionischen oder nichtionischen Polymeren mit geladenen, d. h. anionischen oder kationischen, oder ungeladenen, d. h. nichtionischen Tensiden, können "maßgeschneiderte" Polymerkapseln entstehen, die eine exakte, für die gewünschte Anwendung benötigte Ladung bzw. Substantivität aufweisen.

Die erfindungsgemäß herstellbaren Polymerkapseln können Träger einer Oberflächenladung sein. Daher können die Polymerkapseln auch ein Zeta-Potential aufweisen. Das Zeta-Potential ist das nach außen wirksame Potential der Teilchen, das für deren elektrokinetische Erscheinungen verantwortlich ist und deshalb auch elektrokinetisches Potential genannt wird. Das Zeta-Potential läß sich insbesondere durch mikroskopische Beobachtung der elektrophoretischen Wanderung suspendierter Teilchen bestimmen. Im allgemeinen ist dieses Zeta-Potential im in wäßrigen Medien dispergierten Zustand größer als 10 mV, vorzugsweise größer 20 mV, besonders bevorzugt größer als 30 mV. Diese Oberflächenladung kann für die Substantivität der erfindungsgemäß herstellbaren Polymerkapseln auf Substraten, wie beispielsweise Fasern von Kleidung, vorteilhaft sein.

Erfindungsgemäß erfolgt die Herstellung der duftaktiven Polymerkapseln durch das sogenannte Fällungsverfahren. Dieses Verfahren zur Herstellung von duft- und/oder riechstoffbeladenen Matrix- und/oder Depotsystemen in Form von Polymerkapseln, die mindestens eine duftaktive Komponente enthalten, wie zuvor beschrieben, ist durch die bereits genannten Verfahrensschritte gekennzeichnet.

Hierbei kann die unter (a) hergestellte Mischung, insbesondere unter Rühren und/oder gegebenenfalls unter Erwärmung, langsam in Wasser oder in die wäßrige Lösung eingetragen werden. Vorzugsweise erfolgt das Eintragen der unter (a) hergestellte Mischung unter Vertropfen. Dadurch wird erreicht, daß sich ein feindisperser Niederschlag der erfindungsgemäßen Polymerkapseln bildet. Erfolgt die Zugabe der unter (a) hergestellten Mischung in Wasser oder die wäßrige Lösung zu schnell, so fallen die Polymerkapseln als große, zusammenhängende plastische Masse oder in Form größerer Partikel oder Flocken aus.

Gemäß einer besonders bevorzugten Variante der Erfindung kann bei dem erfindungsgemäßen Fallungsverfahren wie folgt verfahren werden: Ein Duftstoff wird zusammen mit einem Polymer in einem wassermischbaren organischen Lösemittel, z. B. Alkohol oder Aceton, gelöst. Das Polymer ist dabei nicht wasserlöslich. Diese Lösung wird nun in eine ausreichende Menge Wasser eingetragen, wobei das Polymer ausfällt und den Duftstoff einschließt. Die entstehende Dispersion ist, gegebenenfalls unter Zusatz von Hilfsstoffen oder weiteren Filmbildnern, verfilmbar oder kann anderweitig weiterverarbeitet werden. Voraussetzung für dieses Verfahren ist, daß das Polymer beim Eintrag in das Fällungsmittel feindispers ausfällt. Dies ist nur bei sehr wenigen Polymeren der Fall, nämlich bei solchen, die in einem solchen Ausmaß ionische Gruppen tragen, daß sie zwar wasserunlöslich sind, aber dennoch eine elektrostatische Abstoßung der Präzipitatkeime erzeugen, so daß ein feindisperser Niederschlag entsteht. Ein Beispiel ist das zuvor genannte Eudragit^{®} RS oder RL (Fa. Röhm). Die meisten Polymere fallen bei der Fällung in Wasser jedoch als große, zusammenhängende plastische Masse oder in Form größerer Partikel oder Flocken aus und sind daher als solche allein nicht einsetzbar. Erfindungsgemäß werden dem System ionische Tenside zugesetzt. Diese Tenside können dem wäßrigen Fällbad zugesetzt werden und/oder der organischen Mischung bzw. Dispersion, sofern sie darin löslich sind. Die Tenside bewirken eine elektrostatische Abstoßung der bei der Fällung entstehenden Keime und bewirken dadurch einen feindispersen Niederschlag. Durch den erfindungsgemäßen Zusatz von Tensid(en) erhöht sich somit die Anzahl der für dieses Verfahren geeigneten Polymere enorm. In Gegenwart ausreichender Konzentrationen ionischen Tensids sind grundsätzlich alle wasserunlöslichen, aber in wassermischbaren Lösemitteln löslichen Polymere einsetzbar. Geeignet sind z. B. wasserunlösliche Celluloseether und -ester wie Ethylcellulose, Nitrocellulose, Celluloseacetat, Celluloseacetatbutyrat, Polyvinylacetat, wasserunlösliche Vinylacetat/Vinylpyrrolidon-Copolymere sowie diverse Acrylat-oder Methacrylat-Polymere wie z. B. Ultrahold^{®} 8 (Acrylat/Acrylamid-Copolymer) der Fa. BASF. Auch polyurethanbasierte Polymere können ohne weiteres eingesetzt werden, sofern sie die richtige Löslichkeitscharakteristik aufweisen. Weitere Beispiele für geeignete Polymere, d. h. solche, die in Wasser unlöslich, in einem wassermischbaren Lösemittel jedoch löslich sind, finden sich in der folgenden Aufzählung. (A), (E), (T) bzw. (ES) bezeichnet dabei das wassermischbare organische Lösemittel in dem die Polymere löslich sind: Aceton (A), Ethanol (E), Tetrahydrofuran (T) bzw. Essigsäure (ES):
- Poly-1,3-butadiene (T),
- Poly-butadiene-co-acrylnitrile (A),
- Polyisobutene (T),
- allgemein Polyacrylate (T), außer z. B. Poly(2-hydroxyethylacrylate), das wasserlöslich ist,
- viele Polymethacrylate (T), z. B. isotaktisches und syndiotaktisches Polymethylmethacrylate, (T,A),
- Polydimethylitakonate (A,T),
- Polydiamylfumarate (T),
- Polybenzylvinylether (A),
- Polyvinylalkoholacetyle (A),
- Polyallylalcohol (T),
- Polyvinylformale (ES),
- Polyvinylbutyralacetale (E),
- Poly(2-vinyl-4,7-dihydro-1,3-dioxepine) (A),
- Polyvinylmethylketone (A, ES, T),
- Polymethylisopropenylketone (A),
- Polyvinylpivalate (A),
- Polyvinylacetylacetate (ES, T),
- ataktische Polystyrene (T),
- Poly(p-formylstyrene) (T),
- Poly(2-vinyl pyridine) (A),
- Polydimethylfulvene (A),
- Poly(carbonyl-1-furfuryltrimethylene) (A),
- Polytetryhydrofuran (T),
- Polyglutardialdehyde (T),
- Poly(oxycarbonyloxyhexamethylene) (A),
- Polyethylenadipate (T),
- Polymilchsäuren (A),
- Poly(oxymethylhexadecylsilylene) (T).

In Aceton und Alkoholen, wie beispielsweise Ethanol, lösliche Polymere sind gegenüber Polymeren, die in Tetrahydrofuran oder Essigsäure löslich sind, bevorzugt.

Bei dem erfindungsgemäßen Verfahren kann in Schritt (a) die Bereitstellung der Mischung dadurch erfolgen, daß das Polymer und die duftaktive Komponente dem organischen Löse-oder Dispersionsmittel zugesetzt wird. Dieses Zusetzen kann vorzugsweise unter Rühren und gegebenenfalls unter Erwärmung mit der Maßgabe erfolgen, daß möglichst eine homogene Mischung entsteht.

Bei dem erfindungsgemäßen Verfahren kann die gegebenenfalls durchgeführte Abtrennung der duftbeladenen Polymerkapseln durch übliche, dem Fachmann an sich bekannte Methoden, erfolgen. Insbesondere handelt es sich hierbei um Sprühtrocknung unter schonenden Bedingungen, Ultrafiltration, Dialyse oder Gefriertrocknung (Lyophilisation). Im Fall größerer Partikel kann eine Abtrennung auch durch Zentrifugation erreicht werden.

Das in dem erfindungsgemäßen Verfahren verwendete Tensid (grenzflächenaktive Substanz) ist ein ionisches Tensid. Entscheidend ist jedoch, daß die resultierenden Polymerkapseln eine Oberflächenladung aufweisen. Diese Ladung verhindert eine Agglomerisation der Polymerkapseln untereinander während dem erfindungsgemäßen Herstellungsverfahren. Diese Ladung kann durch die Verwendung von geladenen Tensiden erreicht werden; alternativ ist auch die Verwendung von geladenen Polymeren möglich. Allerdings besteht bei Verwendung von entgegengesetzt geladenen Bestandteilen, also z. B. Polymer oder Tensid, die Gefahr, daß es bei dem Herstellungsverfahren zu einer Komplexierung der Bestandteile kommt, woraus die Bildung von größeren Partikeln resultiert. Daher ist die Verwendung von gleichgeladenen Tensiden bzw. Polymeren bevorzugt. Aber alternativ kann auch eine Umladung von geladenen Polymeren durch entgegengesetzt geladene Tenside erfolgen; dadurch ist die Bildung von "maßgeschneiderten" Polymerkapseln möglich.

Wird in dem erfindungsgemäßen Verfahren ein anionisches Tensid verwendet, so kann dieses z. B. ausgewählt sein aus der Gruppe von Seifen; Alkylbenzolsulfonaten; Alkansulfonaten; Olefinsulfonaten; Alkylethersulfonaten; Glycerinethersulfonaten; α-Methylestersulfonaten; Sulfofettsäuren; Alkylsulfaten wie Natriumdodecylsulfat (SDS); Fettalkoholethersulfaten; Glycerinethersulfaten; Fettsäureethersulfaten; Hydroxymischethersulfaten; Monoglycerid(ether)sulfaten; Fettsäureamid(ether)sulfaten; Mono- und Dialkylsulfosuccinaten; Mono- und Dialkylsulfosuccinamaten; Sulfotriglyceriden; Amidseifen; Ethercarbonsäuren und deren Salzen; Fettsäureisothionaten; Fettsäuresarcosinaten; Fettsäuretauriden; N-Acylaminosäuren wie Acyllactylaten, Acyltartraten, Acylglutamaten und Acylaspartaten; Alkyloligoglucosidsulfaten; Proteinfettsäurekondensaten, insbesondere pflanzlichen Produkten auf Weizenbasis; Alkyl(ether)phosphaten. Wird jedoch ein kationisches Tensid verwendet, so kann dieses z. B. ausgewählt sein aus der Gruppe von quartären Ammoniumverbindungen wie Dimethyldistearylammoniumchlorid (CTMA-CI), Dodecyltrimethylammoniumbromid, Didodecyldimethylammoniumbromid, Tridodecylmethylammoniumbromid, Tetradodecylammoniumbromid; Esterquats, insbesondere quaternierten Fettsäuretrialkanolaminestersalzen; Salzen langkettiger primärer Amine quaternären Ammoniumverbindungen wie Hexadecyltrimethyammoniumchlorid; Cetrimoniumchlorid oder Lauryldimethylbenzylammoniumchlorid. Insbesondere bei der Verwendung von ionischen Polymeren kann ein nichtionisches Tensid in den Polymerkapseln enthalten sein; in diesem Fall kann das nichtionische Tensid z. B. ausgewählt sein aus der Gruppe von (i) nichtpolymeren nichtionischen Tensiden wie alkoxylierten, vorzugsweise ethoxylierten Fettalkoholen, Alkylphenolen, Fettaminen und Fettsäureamiden; alkoxylierten Triglyceriden, Mischethern und Mischformalen; gegebenenfalls partiell oxidierten Alk(en)yloligoglykosiden; Giucoronsäurederivaten; Fettsäure-N-alkylglucamiden; Proteinhydrolysaten, insbesondere alkylmodifizierten Proteinhydrolysaten; niedermolekularen Chitosanverbindungen; Zuckerestern; Sorbitanestern; Aminoxiden; und (ii) polymeren nichtionischen Tensiden wie Fettalkoholpolyglykolethern; Alkylphenolpolyglykolethern-, Fettsäurepolyglykolestern; Fettsäureamidpolyglykolethern; Fettaminpolyglykolethern; Polyolfettsäureestem; Polysorbaten.

Das in dem erfindungsgemäßen Verfahren verwendete Polymer kann ein nichtionisches oder ein ionisches Polymer sein. Gleichfalls kann das Polymer ein geblocktes und/oder statistisches Comonomer umfassen. Insbesondere kann das Polymer ausgewählt sein aus der Gruppe von Cellulosen und Cellulosederivaten wie Ethylcellulosen, Celluloseacetaten und Celluloseacetatbutyraten, Polyalkylenen wie Polybutadienen, Poly(butadien-co-acrylnitrilen), Polyisobutenen, Polyamiden, Polystyrolen, Polyisoprenen, Polycarbonaten, Polyestern, Polyacrylaten, Polymethacrylaten, insbesondere ammoniumfunktionalisierten Polymethacrylaten, Polyurethanen, Polydimethylitakonaten, Polydiamylfumaraten, Polybenzylvinylethern, Polyvinylalkoholen, Polyallylalkoholen, Polyvinylformalen, Polyvinylbutyraten, Poly(2-vinyl-4,7-dihydro-1,3-dioxepinen), Polyvinylmethylketonen, Polymethylisopropylketonen, Polyvinylpivalaten, Polyvinylacetylacetaten, Poly(p-formylstyrolen), Poly(2-vinylpyridinen), Polydimethylfulvenen, Poly(carbonyl-1-furfuryltrimethylenen), Polytetrahydrofuranen, Poly(glutardialdehyden), Polyoxycarbonyloxyhexamethylenen, Polyethylenadipaten, Polyvinylalkoholacetaten, Polyvinylbutyralacetalen, Polymilchsäuren und Polyoxymethylhexadecylsilylenen.

Die im erfindungsgemäßen Verfahren verwendeten Polymere und/oder duftaktiven Komponenten sollten vorzugsweise im wesentlichen wasserunlöslich oder zumindest in der wäßrigen Phase nur schwer löslich sein. Vorzugsweise sind die Polymere und/oder die duftaktive(n) Komponente(n) in einem solchen Fall in der wäßrigen Phase zu weniger als 10 %, vorzugsweise zu weniger als 5 %, insbesondere zu weniger als 1 %, löslich.

Die mittlere Teilchengröße der durch das erfindungsgemäße Verfahren erhältlichen Polymerkapseln kann in großen Bereichen schwanken: Sie beträgt im allgemeinen etwa 50 nm bis etwa 500 µm, insbesondere etwa 100 nm bis etwa 250 µm, vorzugsweise etwa 200 nm bis etwa 100 µm. Eine Steuerung der Teilchengröße ist durch Variation der Verfahrensparameter möglich (z. B. Variation des Tensidgehalts, der Gesamtkonzentration der Lösung bzw. Dispersion, Art und Konzentration des Polymers, Art und Konzentration der Duftkomponente etc.). Die Teilchengröße kann zusätzlich insbesondere durch die Tröpfchengröße und die Zutropfgeschwindigkeit im Verfahrensschritt (b) gesteuert werden.

Im erfindungsgemäßen Verfahren kann das Löse- oder Dispersionsmittel das Tensid darstellen bzw. umfassen. Dieses ist beispielsweise bei der Verwendung von Ölsäure als Löse- bzw. Dispersionsmittel und gleichzeitig als Tensid der Fall.

Die Erfindung ermöglicht Dispersionen, vorzugsweise wäßrige Dispersionen, welche die erfindungsgemäß herstellbaren Polymerkapseln enthalten. Der Gehalt an Polymerkapseln in diesen erfindungsgemäßen Dispersionen kann in weiten Bereichen variieren: Er beträgt im allgemeinen bis zu 20 %, insbesondere bis zu 15 %, vorzugsweise bis zu 10 %. Dabei sind die Gewichtsangaben auf die Dispersion bezogen. Die erfindungsgemäß herstellbaren Dispersionen können z. B. in Form von Wasch- und Reinigungsmitteln, Kosmetika oder Körperpflegemittel verwendet werden. Im Fall wäßrige Dispersionen können diese variierende Anteile an organischem Löse- und Dispersionsmittel (insbesondere aus dem Herstellungsverfahren) enthalten, wobei jedoch ein Wasseranteil, bezogen auf die flüssige Phase, von 50% nicht überschritten werden sollte.

Die erfindungsgemäß herstellbaren Polymericapseln sowie die erfindungsgemäß herstellbaren Dispersionen können in der Waschmittel- und Reinigungsmittelindustrie bzw. in der Kosmetik- und Körperpflegeindustrie verwendet werden. Die Verwendung kann dabei beispielsweise als Delivery-System erfolgen, wobei die duftaktive(n) Komponente(n) insbesondere mit verlängerter oder verzögerter Freisetzung wirkt/wirken (sogenannter "Sustained-release-Effekt").

Die erfindungsgemäß herstellbaren Polymerkapseln und Dispersionen können auch zur kontrollierten bzw. verzögerten Freisetzung von duftaktiven Komponenten verwendet werden.

Ein weitere Verwendung bzw. Anwendung der erfindungsgemäß herstellbaren Polymerkapseln und Dispersionen ist ihre Auftragung als Beschichtung, Film bzw. Überzug. Dabei kann die Steuerung der Freisetzung der duftaktiven Komponente durch die Auswahl der Art der Zusammensetzung der Polymerkapseln, insbesondere durch die Auswahl der Art des Polymers und der Duftkomponente(n), erfolgen. Die Auftragung kann beispielsweise zur Herstellung von Trägeroberflächen mit Depothaltung von Duftkomponenten verwendet werden und dient somit zum Schutz und/oder zur Depothaltung von Duft- und/oder Riechstoffen, Duft- und/oder Riechstoffzubereitungen und Ölen wie etherischen Ölen, Aromaölen, Parfümölen, Pflegeölen und Silikonölen auf diesen Trägeroberflächen. Diese erfindungsgemäß herstellbaren Kapsel- bzw. Dispersionssysteme können sowohl auf harten Oberflächen verfilmt werden, in weitere Filmbildner eingebettet werden oder auch, gegebenenfalls unter Zusatz weiterer Hilfsstoffe, auf Textilien aufgezogen werden, so daß sie einen länger anhaltenden Dufteindruck erzeugen. Erfindungsgemäß versteht man unter dem Begriff "Film" nicht unbedingt eine zusammenhängende, geschlossene Schicht, sondern eine Verteilung der erfindungsgemäß herstellbaren Polymerkapseln auf der Oberfläche, wobei diese Verteilung auch unterbrochen sein kann. Möglich sind somit auch Filme, Überzüge, Schichten und/oder Beschichtungen, die ausgehend von den erfindungsgemäßen Polymerkapseln oder Dispersionen erhalten werden können.

Möglich sind auch Oberflächen, insbesondere Oberflächen von inerten Trägermaterialien, auf die Filme, Überzüge, Schichten, Beschichtungen oder dergleichen der zuvor genannten Art aufgebracht sind. Wie zuvor beschrieben, handelt es sich hierbei insbesondere um Oberflächen mit Depotfunktion für duftaktive Komponenten bzw. mit kontrollierter und/oder verzögerter Freisetzungsfunktion für duftaktive Komponenten, wie Duft- und/oder Riechstoffe, Duft- und/oder Riechstoffzubereitungen und Öle wie etherische Öle, Aromaöle, Parfümöle, Pflegeöle und Silikonöle.

Mit der vorliegenden Erfindung ist eine Vielzahl von Vorteilen verbunden. Um nur einige zu nennen, seien beispielhaft die folgenden genannt: Die Erfindung stellt ein mit sehr geringem technischen Aufwand durchführbares Verfahren zur Herstellung duftaktiver Polymerkapsein und Beschichtungen dar, welches die erfindungsgemäßen Dispersionen in verwendungsfertiger Form bereitstellt. Die erfindungsgemäßen Verfahren liefern also - neben den Polymerkapseln selbst - zugleich auch wäßrige Dispersionen, die sofort verwendet werden können. Die Verwendung dieser Polymerkapseln in Wasch-, Reinigungs- oder Nachbehandlungsmitteln sowie Kosmetika führt zu einem verlängerten Dufteindruck gegenüber der Dosierung des reinen Parfümöls. Die Verwendung in Reinigern, insbesondere Bodenreinigem, führt zu einem duftaktiven Film auf den Substraten und damit zu einem verlängerten Dufteindruck in den gereinigten Räumen. Im Gegenzug ist der Dufteindruck unmittelbar nach der Anwendung nicht so intensiv. Daher sinkt die Gefahr, daß er vom Verbraucher zunächst als zu stark und damit als zu unangenehm empfunden wird. Ferner bieten sich die erfindungsgemäßen Dispersionen insbesondere zum leichteren Dosieren von duftaktiven Verbindungen an, insbesondere zur Formulierung von Wasch- und Reinigungsmitteln bzw. im Bereich der Kosmetik- und Körperpflegeindustrie. So müssen diese Dispersionen nicht durch nachträgliches Dispergieren von Polymerkapseln in Wasser oder wäßrigen Lösungen hergestellt werden.

Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne daß er dabei den Rahmen der vorliegenden Erfindung verläßt.

Die vorliegende Erfindung wird anhand der folgenden Ausführungsbeispiele veranschaulicht, welche die Erfindung jedoch keinesfalls beschränken.

### Ausführungsbeispiele:

### Beispiel 1: Verfahren zur Herstellung von riechstoffhaltigen Mikropartikeln

Es wird eine organische Phase aus Ethylacetat mit 0,1 Gew.% n-Decan hergestellt, in die 5 Gew.% wasserunlösliches Celluloseacetatbutyrat-Polymer sowie 1 Gew.-% Parfümöl gelöst werden. In die vorgelegte organische Phase wird unter Ultraturrax^{®}-Behandlung die dreifache Menge einer wäßrigen Emulgatorlösung von 0,5 Gew.-% Dodecyltrimethylammoniumbromid eindispergiert. Es entsteht eine feindisperse Emulsion mit der wäßrigen Phase als äußere, kohärente Phase. Aus dieser Emulsion wird unter Vakuum das leichter flüchtige Lösemittel langsam abgezogen. Es verbleibt eine feindisperse, milchig-weiße Dispersion mit Partikelgrößen von weniger als 1 µm. In den Partikeln befinden sich also 16,6 % Parfümöl, der Gesamtgehalt an Parfüm in der Dispersion beträgt 0,33 %.

### Beispiel 2: Gefällte Polyacrylat-Partikel (nicht erfindungsgemäß)

9,25 g einer 10%igen ethanolischen Lösung (wt/wt) von Eudragit RS^{®} (Fa. Röhm) werden mit 0,75 g Parfümöl vermischt. Die Lösung wird unter Rühren in 90 g Wasser eingetragen. Dabei fallen feindisperse Polymerpartikel (< 1 µm) mit einem (berechneten) Parfümgehalt von ca. 45 % in den Partikeln bzw. 0,75 % in der gesamten Dispersion aus.

Wegen der kationischen Ladung der Partikel eignen sich diese Polymerkapseln insbesondere für eine Verwendung in Weichspülerrezepturen.

### Beispiel 3: Anionisch stabilisierte, gefällte Ethylcellulose-Partikel

Es wird eine 1 %ige acetonische Lösung (wt/wt) von Ethylcellulose (Typ EC N7, Fa. Hercules) hergestellt, in der 0,3 % Parfümöl gelöst werden. Die Lösung wird unter Rühren in die zehnfache Menge eines tensidhaltigen wäßrigen Fällbades eingetragen (0,02 % Natriumdodecylsulfat (SDS) in Wasser (wt/wt)). Dabei fällt die Ethylcellulose als feindisperser, riechstoffhaltiger Niederschlag aus (Partikelgrößen < 1 µm).

Die so hergestellten, anionisch stabilisierten Partikel eignen sich insbesondere zur Formulierung in anionisch basierten Reinigerrezepturen, wie in Beispiel 12 beschrieben. Sie können entweder durch Zentrifugation oder Filtration isoliert werden oder aber auch direkt z. B. zu einem Film weiterverarbeitet werden.

Die so erhaltene, feinteilige EC-Dispersion wird auf einem Glassubstrat durch Ausrakeln aufgetragen. Nach Eintrocknen des Lösemittels bleibt ein kohärenter Film zurück, der auch nach einigen Tagen noch eine Duftwirkung aufweist. Für die Praxis relevanter ist jedoch die im Beispiel 12 beschriebene Verarbeitung in einer Rezeptur für Reiniger für harte Oberflächen.

### Beispiel 4: Kationisch stabilisierte, gefällte Ethylcellulosepartikel

Es wird eine 1 %ige acetonische Lösung (wt/wt) von Ethylcellulose (Typ EC N7, Fa. Hercules) hergestellt, in der 0,6 % Parfümöl gelöst werden. Diese Lösung wird unter Rühren in die gleiche Menge einer 0,05%igen wäßrigen Lösung von Dehyquat^{®} A (Fa. Cognis) eingetragen. Auch hier entsteht ein feindisperser Niederschlag.

Die so hergestellten, kationisch stabilisierten Partikel eignen sich insbesondere zur Formulierung in kationisch basierten Rezepturen für Textilnachbehandlungsmittel, z. B. Weichspüler, wie in den Beispielen 8 bis 10 beschrieben. Sie können entweder durch Zentrifugation oder Filtration abgetrennt werden, aufkonzentriert oder aber auch direkt weiterverarbeitet werden.

### Beispiel 5: Kationisch stabilisierte, gefällte riechstoffhaltige Acrylatpartikel

Es wird eine ethanolische Lösung von 1 % des Acrylat-Polymers Ultrahold^{®} 2 (Fa. BASF), 1 % Didodecyldimethylammoniumbromid (DDMABr) und 0,4 % Parfümöl hergestellt. Ein Teil dieser Lösung wird unter Rühren in 9 Teile Wasser eingetragen. Man erhält einen feindispersen Niederschlag aus Polymerpartikeln mit einem Gehalt von 16,6 % Parfümöl. Wie bei den vorigen Beispielen können die parfümhaltigen Mikropartikel durch Zentrifugation oder Filtration gewonnen werden oder aber direkt weiterverarbeitet werden. Infolge der kationischen Stabilisierung eignet sich diese Dispersion besonders zur Weiterverarbeitung in Weichspülern.

### Beispiel 6: Gefällte riechstoffhaltige Polyvinylacetatpartikel

Es wird eine ethanolische Lösung hergestellt, die 1 % Polyvinylacetat (Fa. Aldrich), 1 % Didodecyldimethylammoniumbromid (DDMABr) und 0,5 % Parfümöl enthält. Ein Teil dieser Lösung wird unter Rühren in 9 Teile Wasser eingetragen, wobei ein feinteiliger Niederschlag aus Partikeln mit einem Gehalt von 20 % Parfümöl ausfällt. Wie bei den vorigen Beispielen können die parfümhaltigen Mikropartikel durch Zentrifugation oder Filtration gewonnen werden oder aber direkt weiterverarbeitet werden. Infolge der kationischen Stabilisierung eignet sich diese Dispersion besonders zur Weiterverarbeitung in Weichspülem.

### Beispiel 7: Geruchliche Beurteilung

Für die in den folgenden Beispielen erwähnte geruchliche Beurteilung von harten Oberflächen, Räumen oder Textilien wird jeweils ein Blindversuch mit einem zehn Personen umfassenden Panel durchgeführt. Jedes der Panelmitglieder beurteilt die Duftintensität der erfindungsgemäßen Formulierungen gegen die des Vergleichsbeispiels. Die in den Beispielen angegebenen Ergebnisse geben jeweils die Meinung der Mehrheit der Panelmitglieder wieder. In der überwiegenden Anzahl der Versuche war das Ergebnis sehr eindeutig.

### Beispiel 8: Verwendung der Partikel aus Beispiel 1 in einer Weichspülerrezeptur

In 100 ml der Dispersion aus Beispiel 2 (entsprechend 0,33 g Parfümöl) werden 5,4 g Esterquat (Stepantex^{®} VL 90) eingearbeitet, indem die Dispersion auf 40 °C erwärmt wird und der ebenfalls auf 40 °C temperierte Esterquat unter Rühren zugegeben wird.

### Vergleich:

5,4 g Esterquat werden, wie oben beschrieben, in 100 ml Wasser dispergiert. Anschließend wird dieser Dispersion 0,33 g Parfümöl zugesetzt.

Diese Weichspülerrezepturen werden anschließend in einer Waschmaschine getestet: 100 ml der jeweiligen Rezepturen werden in die Weichspüler-Einspülkammern gegeben, so daß sie im letzten Spülgang dosiert werden. Die Trommelfüllung besteht aus 3,5 kg Frotteehandtüchern.

Nach der Entnahme der Wäsche aus der Maschine werden von dem Versuchspanel die mit der Rezeptur des Vergleichsbeispiels gespülten Textilien zunächst als intensiver duftend eingestuft. Nach einem Tag und an jedem weiteren Tag jedoch ist der Duft, der mit der erfindungsgemäßen Rezeptur weichgespülten Wäsche eindeutig intensiver als beim Vergleichsbeispiel.

### Beispiel 9: Verwendung der Partikel aus Beispiel 2 in einer Weichspülerrezeptur

In 100 ml der Dispersion aus Beispiel 2 (entsprechend 0,75 % Parfümöl) werden 5,4 g Esterquat (Stepantex^{®} VL 90) eingearbeitet, indem die Dispersion auf 40 °C erwärmt wird und der ebenfalls auf 40 °C temperierte Esterquat unter Rühren zugegeben wird.

### Vergleich:

5,4 g Esterquat werden, wie oben beschrieben, in 100 ml Wasser dispergiert. Anschließend wird dieser Dispersion 0,75 g Parfümöl zugesetzt.

Diese Weichspülerrezepturen werden anschließend wie in Beispiel 8 in einer Waschmaschine getestet. 100 ml der jeweiligen Rezepturen werden in die Weichspüler-Einspülkammern gegeben, so daß sie im letzten Spülgang dosiert werden. Die Trommelfüllung besteht wiederum aus 3,5 kg Frotteehandtüchem.

Nach der Entnahme der Wäsche aus der Maschine wird von dem Versuchspanel die mit der Rezeptur des Vergleichsbeispiels gespülten Textilien zunächst als intensiver duftend eingestuft. Nach einem Tag und an jedem weiteren Tag ist der Duft der mit der erfindungsgemäßen Rezeptur weichgespülten Wäsche eindeutig intensiver als beim Vergleichsbeispiel.

### Beispiel 10: Verwendung der Partikel aus Beispiel 4 in einer Weichspülerrezeptur

Die riechstoffhaltige EC-Dispersion aus Beispiel 4 wird zunächst mit Hilfe eines Membranfilters mit 250 nm Porendurchmesser um den Faktor 2 aufkonzentriert. Damit berechnet sich eine Parfümölkonzentration von 0,6 %. Geringe Verluste durch die Filtration sind nicht auszuschließen. In 100 ml dieser Dispersion (entsprechend 0,6 g Parfümöl) werden 5,4 g Esterquat (Stepantex^{®} VL 90) eingearbeitet, indem die Dispersion auf 40 °C erwärmt wird und der ebenfalls auf 40 °C temperierte Esterquat unter Rühren zugegeben wird.

### Vergleich:

5,4 g Esterquat werden, wie oben beschrieben, in 100 ml Wasser dispergiert. Anschließend wird dieser Dispersion 0,6 g Parfümöl zugesetzt.

Der Test erfolgt wie in den Beispielen 8 und 9. Wieder wird nach der Entnahme der Wäsche aus der Maschine von dem Versuchspanel die mit der Rezeptur des Vergleichsbeispiels gespülten Textilien zunächst als intensiver, teilweise unangenehm intensiv duftend eingestuft. Nach einem Tag und an jedem weiteren Tag war der Duft der mit der erfindungsgemäßen Rezeptur weichgespülten Wäsche dann eindeutig intensiver als beim Vergleichsbeispiel.

### Beispiel 11: Verwendung der Partikel aus Beispiel 5 und 6 in einer Weichspülerrezeptur

Diese Versuche werden analog zu Beispiel 10 durchgeführt, wobei die Dispersion mit Hilfe des Membranfilters so aufkonzentriert wird, daß sie (berechnet, ohne Berücksichtigung eventueller Verluste) 0,6 % Parfümöl enthält. Die Versuchsdurchführung sowie das Ergebnis entspricht den Beispielen 8 bis 10.

### Beispiel 12: Verwendung der Partikel aus Beispiel 3 in einer Modellrezeptur eines Reinigers für harte Oberflächen

In diesem Beispiel wird die Verwendung der anionisch stabilisierten Dispersion aus Beispiel 3 in einer Modellrezeptur für einen Reiniger beschrieben. Diese Rezeptur steht modellhaft für einen Bad-, Küchen-, Fußboden- oder Universalreiniger.

Dazu wird die Dispersion aus Beispiel 3 zunächst mit Hilfe eines Membranfilters (220 nm Porendurchmesser) aufkonzentriert, so daß die resultierende Dispersion einen Parfümölgehalt von 0,5 % aufweist. Zur Herstellung der Reiniger-Modellrezeptur werden 92 g dieser Dispersion 5 g lineares Alkybenzolsulfonat (Maranil^{®}, Fa. Cognis) und 3 g Dehydol^{®} LT7 (Fa. Cognis) zugesetzt. Als Vergleichsbeispiel wird einer gleich konzentrierten Tensidlösung 0,46 g Parfümöl zugesetzt. Die Vergleichsrezeptur weist von sich aus einen intensiveren Duft auf als die erfindungsgemäße Rezeptur, wobei der Duft der erfindungsgemäßen Rezeptur noch von einer leichten Aceton-Note überlagert ist.

Zur geruchlichen Beurteilung werden aus diesen Reinigerrezepturen (erfindungsgemäß und Vergleichsbeispiel) durch 10flache Verdünnung ein Wischwasser hergestellt. Mit diesem Wischwasser wird mit Hilfe eines Lappens der PVC-Fußboden zweier Räume mit geringem Luftaustausch gewischt. Im Raum, in dem die Rezeptur des Vergleichsbeispieles angewandt wird, verfliegt der Duft bereits nach kurzer Zeit, spätestens aber nach Durchlüftung. Im Raum, der mit der erfindungsgemäßen Zusammensetzung gewischt wird, ist der Duft unmittelbar nach dem Wischen weniger intensiv, ist aber dann über mehrere Tage hin wahrnehmbar und baut sich auch nach Durchlüften wieder langsam auf.

## Patentansprüche

1. Verfahren zur Herstellung von duft- und/oder riechstoffbeladenen Matrix- und/oder Depotsystemen in Form von Polymerkapseln mit mindestens einer duftaktiven Komponente, **gekennzeichnet durch** die folgenden Verfahrensschritte:
(a) Bereitstellung einer Lösung oder Dispersion, die mindestens ein nicht wasserlöslicher Polymer, welches in wässriger Phase zu weniger als 10% löslich ist, und mindestens eine duftaktive Komponente, ausgewählt aus der Gruppe von Duft- und/oder Riechstoffen, Duft-und/oder Riechstoffzubereitungen und Ölen wie etherischen Ölen, Aromaölen, Parfümölen, enthält, mit einem mit Wasser mischbarem organischen Löse- oder Dispersionsmittel;
(b) Eintragung der unter (a) hergestellten Mischung in Wasser oder in eine wässrige Lösung, in Gegenwart eines ionischen Tensids, so dass man duftbeladene Polymerkapseln in wässriger Dispersion erhält;
(c) gegebenenfalls Abtrennung der auf diese Weise erhaltenen Polymerkapseln, wobei die resultierenden Polymerkapseln eine Oberflächenladung aufweisen.

2. Verfahren nach Anspruch 1, **dadurch gekennzeichnet, dass** in Schritt (a) die Bereitstellung der Mischung dadurch erfolgt, dass das Polymer und die duftaktive Komponente dem organischen Löse- oder Dispersionsmittel unter Rühren, gegebenenfalls unter Erwärmung, zugesetzt werden.

3. Verfahren nach einem der Ansprüche 1 und 2, **dadurch gekennzeichnet, dass** das Löse-oder Dispersionsmittel das Tensid ist und/oder umfasst, insbesondere wobei das Löse-oder Dispersionsmittel und gleichzeitig das Tensid Ölsäure ist.

## Claims

1. Process for the production of matrix systems and/or depot systems loaded with fragrance and/or odoriferous substance in the form of polymer capsules with at least one active fragrance component, **characterised by** the following process steps:
(a) providing a solution or dispersion that comprises at least one water-insoluble polymer that is less than 10% soluble in the aqueous phase, and at least one active fragrant component, selected from the group of fragrances and/or odoriferous substances, preparations of fragrances and/or of odoriferous substances, and oils such as aethereal oils, aromatic oils, perfume oils, with a water-miscible organic solvent or dispersion medium;
(b) introducing the mixture produced under (a) into water or into an aqueous solution in the presence of an ionic surfactant, such that fragrance-loaded polymer capsules are obtained in an aqueous dispersion;
(c) optionally separating the thus obtained polymer capsules, wherein the resulting polymer capsules possess a surface charge.

2. Process according to claim 1, **characterised in that** in step (a) the provision of the mixture takes place such that the polymer and the active fragrance component are added to the organic solvent or dispersion medium with stirring, optionally with heating.

3. Process according to one of claims 1 and 2, **characterised in that** the solvent or dispersion medium is and/or contains the surfactant, in particular wherein the solvent or dispersion medium and simultaneously the surfactant is oleic acid.

## Revendications

1. Procédé de préparation de systèmes à matrice et/ou à dépôt chargés de parfum et/ou de substance odoriférante sous forme de capsules de polymère présentant au moins un composant parfumant actif, **caractérisé par** les étapes de procédé suivantes :
(a) préparation d'une solution ou d'une dispersion qui contient au moins un polymère non soluble dans l'eau, qui est soluble à moins de 10% en phase aqueuse, et au moins un composant parfumant actif, choisi dans le groupe des parfums et/ou des substances odoriférantes, des compositions de parfum et/ou de substances odoriférantes et des huiles, telles que les huiles essentielles, les huiles aromatisées, les huiles parfumées, ainsi qu'un solvant ou un dispersant organique miscible à l'eau ;
(b) introduction du mélange préparé sous (a) dans l'eau ou dans une solution aqueuse, en présence d'un agent tensioactif ionique, de manière à obtenir des capsules de polymère chargées de parfum en dispersion aqueuse ;
(c) le cas échéant séparation des capsules de polymère ainsi obtenues, les capsules de polymère résultantes présentant une charge superficielle.

2. Procédé selon la revendication 1, **caractérisé en ce que** dans l'étape (a), la préparation du mélange a lieu **en ce que** le polymère et le composant actif en parfum sont ajoutés au solvant ou dispersant organique sous agitation, le cas échéant en chauffant.

3. Procédé selon l'une quelconque des revendications 1 et 2, **caractérisé en ce que** le solvant ou le dispersant est et/ou comprend l'agent tensioactif, le solvant ou le dispersant et simultanément l'agent tensioactif étant en particulier l'acide oléique.
